# EUROPÄISCHE PATENTSCHRIFT

(11) **EP 1 455 595 B1**
(45) Veröffentlichungstag und Bekanntmachung des Hinweises auf die Patenterteilung: **07.05.2008**
(21) Anmeldenummer: 02798239.6
(22) Anmeldetag: 19.12.2002
(51) Int. Cl.: A23L 1/03, A23K 1/00, A23L 1/015, A23L 1/105, C12R 1/01

(54) **MIKROORGANISMUS ZUR BIOLOGISCHEN ENTGIFTUNG VON MYKOTOXINEN, NÄMLICH OCHRATOXINEN UND/ODER ZEARALENONEN, SOWIE VERFAHREN UND VERWENDUNG HIEFÜR**
MICROORGANISM FOR THE BIOLOGICAL DETOXIFICATION OF MYCOTOXINS, NAMELY OCHRATOXINS AND/OR ZEARALENONES, AND CORRESPONDING METHOD AND USE
MICRO-ORGANISME POUR LA DECONTAMINATION BIOLOGIQUE DE MYCOTOXINES, NOTAMMENT D'OCHRATOXINES ET/OU DE ZEARALENONES, PROCEDE ET UTILISATION ASSOCIES

(30) Priorität: 20.12.2001 AT 20002001
(43) Veröffentlichungstag der Anmeldung: 15.09.2004
(73) Patentinhaber: Erber Aktiengesellschaft, 3130 Herzogenburg (AT)
(72) Erfinder: SCHATZMAYR, Gerd, A-1190 Wien (AT); HEIDLER, Dian, A-1190 Wien (AT); FUCHS, Elisabeth, 3430 Tulln (AT); BINDER, Eva-Maria, A-7501 Unterwart (AT)
(74) Vertreter: Cunow, Gerda
(86) Internationale Anmeldenummer: PCT/AT2002/000356
(87) Internationale Veröffentlichungsnummer: WO 2003/053161

(56) Entgegenhaltungen:
- WO-A-02/076205
- AT-B- 406 166
- AT-U- 504
- US-A- 4 004 978

## Beschreibung

Die vorliegende Erfindung bezieht sich auf einen Mikroorganismus zur biologischen Entgiftung von Mykotoxinen, nämlich Ochratoxinen und/oder Zearalenonen, sowie auf ein Verfahren zur biologischen Entgiftung von Mykotoxinen, nämlich Ochratoxinen und/oder Zearalenonen, in Nahrungs- und Futtermitteln mit wenigstens einem Mikroorganismus sowie auf die Verwendung von Bakterien und/oder Hefen zur Detoxifizierung von Ochratoxinen und/oder Zearalenonen in Nahrungs- oder Futtermitteln.

Mykotoxine sind natürliche, sekundäre Stoffwechselendprodukte von Schimmelpilzen, die weltweit landwirtschaftliche Produkte befallen und schon in geringen Mengen toxische Wirkungen hervorrufen. Aufgrund des Befalls von landwirtschaftlichen Produkten mit Mykotoxinen entstehen extrem hohe Schäden und es werden weiters Mykotoxikosen bei Menschen und Tieren hervorgerufen, welche teilweise dramatische Wirkungen zeigen. Aufgrund der hohen wirtschaftlichen Verluste sowie der Belastung von Menschen und Tieren mit Mykotoxinen und den dadurch hervorgerufenen Mykotoxikosen wurde bereits seit langem nach Möglichkeiten gesucht, Maßnahmen gegen die Kontaminierung mit Mykotoxinen zu ergreifen, wobei aus der Literatur prinzipiell zwei Methoden bekannt sind. Eine erste versucht, das Wachstum der Schimmelpilze auf Lebens- bzw. Futtermitteln zu verhindern, wodurch gleichzeitig die Produktion der Mykotoxine verhindert wird. Eine zweite Methode versucht, nachträglich die Mykotoxine zu zerstören oder Lebens- und/oder Futtermittel zu dekontaminieren.

So ist beispielsweise in der WO 91/13555 ein Futtermittelzusatz sowie ein Verfahren zur Inaktivierung von Mykotoxinen beschrieben, wobei dem Futter Teilchen eines Phyllosilikatminerals zugesetzt werden, um die Mykotoxine zu inaktivieren. Zur Verstärkung der Wirkung dieser Phyllosilikate sind die Teilchen mit einem Sequestrierungsmittel beschichtet, um die Wirkung zu beschleunigen. Weiters ist beispielsweise aus der WO 92/05706 ein Futtermittel bekannt geworden, in welchem Montmorillonit-Ton als Futtermittelzusatz enthalten ist. Diese natürlichen Tonmineralien mit großer innerer Oberflächen sollen aufgrund ihrer Porosität die Mykotoxine oberflächlich binden und auf diese Weise immobilisieren.

Weiters ist aus dem österreichischen Gebrauchsmuster AT-U 504 ein Futtermittelzusatz bekannt geworden, in welchem eine Enzymzubereitung verwendet wird, welche fähig ist, Epoxidasen und Laktonasen zu bilden und Mykotoxine chemisch sowohl im Futtermittel als auch im Magen-Darm-Trakt von Tieren abzubauen. Gemäß der AT-U 504 kann die Wirkung dieser Enzymzubereitung durch den Zusatz von Zeolithen und dgl. verstärkt werden.

Die Zugabe von Mykotoxinbindern zu Futtermitteln, die im Zuge der Verdauung unmittelbar im Verdauungstrakt eine Bindung mit den Mykotoxinen eingehen, können die Effekte der Toxine bei der Nutztierhaltung minimieren. Neben den oben erwähnten Möglichkeiten zählen Alfalfa, Bentonite, Zeolith, Tone, Aktivkohle, hydrierte Natriumcalciumaluminiumsilikate, Phyllosilikate und Hefe- oder Bakterienzellwände (US 5,165,946; WO 99/57994; US 6,045,834; EP 0721741; US 5,165,946; US 5,935,623; WO 98/34503; WO 00/41806) zu den eingesetzten Substanzen. Die Bindung der Toxine an diese Materialien ist abhängig von den Struktureigenschaften der Toxine. So ist bis heute noch kein effektiver Mykotoxinbinder für die Trichothecene gefunden worden. Ein weiterer Nachteil der Mykotoxinbinder ist, daß sie neben den Toxinen auch wichtige Nährstoffe, wie Vitamine oder Antibiotika, aus den Futtermitteln adsorbieren können.

Kürzlich wurde festgestellt, daß Mykotoxine durch Mikroorganismen abgebaut und somit teilweise detoxifiziert werden können. Ein Beispiel für die Detoxifizierung von Mykotoxinen, insbesondere Trichothecenen, ist in der AT-B 406 166 enthalten, bei welchem eine spezielle Reinkultur eines zum Genus Eubacterium gehörenden Mikroorganismus, welcher unter der Nummer DSM 11798 hinterlegt wurde sowie einer Mischkultur des Genus Eubacterium mit einem Enterococcus, welcher unter der Nummer 11799 hinterlegt wurde, die Trichothecene durch Spaltung des an Trichothecenen vorhandenen Epoxyringes detoxifizieren.

Aus US-A 4,004,978 ist die Verwendung von Rhodotorula oder einem entsprechenden Rohextrakt bzw. zellfreien Extrakt aus Rhodotorula für den Abbau von Zearalenonen bekannt geworden. Ähnliches ist aus der nachveröffentlichten WO-A-02/076205 zu entnehmen.

Die Detoxifizierung von Ochratoxinen durch enzymatische Hydrolyse wurde bereits von M. J. Pitout: The hydrolysis of ochratoxin A by some proteolytic enzymes, Biochem. Pharmacol. 18, 485-491 (1969), beschrieben.

Die vorliegende Erfindung zielt nun darauf ab, spezielle Mikroorganismen sowie Misch- oder Reinkulturen und auch Kombinationen derselben zur Verfügung zu stellen, mit welchen es gelingt, Mykotoxine, nämlich Ochratoxine und/oder Zearalenone, gezielt biochemisch abzubauen und in physiologisch unbedenkliche und insbesondere in der Futtermittel- und Nahrungsmittelindustrie unbedenkliche Substanzen umzuwandeln.

Zur Lösung dieser Aufgaben ist der erfindungsgemäße Mikroorganismus der eingangs genannten Art im wesentlichen dadurch gekennzeichnet, daß ein Mikroorganismus, insbesondere aerobe oder anaerobe, detoxifizierende Bakterien oder Hefen, eingesetzt ist bzw. sind, welche(s) die Phenylalaningruppe der Ochratoxine abspalten bzw. Zearalenone abbauen, wobei die Mikroorganismus detoxifizierenden Bakterien aus Sphingomonas sp. DSM 14170 und DSM 14167, Stenotrophomonas nitritreducens DSM 14168, Stenotrophomonas sp. DSM 14169, Ralstonia eutropha DSM 14171 und Eubacterium sp. DSM 14197 gewählt sind, und/oder die detoxifizierenden Hefen aus Trichosporon spec. nov. DSM 14153, Cryptococcus sp. DSM 14154, Rhodotorula yarrowii DSM 14155, Trichosporon mucoides DSM 14156 und Trichosporon dulcitum DSM 14162 gewählt sind. Dadurch, daß ein Mikroorganismus, insbesondere aerobe oder anaerobe, detoxifizierende Bakterien oder Hefen, gewählt aus Sphingomonas sp. DSM 14170 und DSM 14167, Stenotrophomonas nitritreducens DSM 14168, Stenotrophomonas sp. DSM 14169, Ralstonia eutropha DSM 14171 und Eubacterium sp. DSM 14197, sowie die detoxifizierenden Hefen, gewählt aus Trichosporon spec. nov. DSM 14153, Cryptococcus sp. DSM 14154, Rhodotorula yarrowii DSM 14155, Trichosporon mucoides DSM 14156 und Trichosporon dulcitum DSM 14162, eingesetzt ist bzw. sind, welche(s) die Phenylalaningruppe der Ochratoxine abspalten bzw. Zearalenone abbauen, gelingt es, Ochratoxine, insbesondere Ochratoxin A oder Ochratoxin B, in jene Metaboliten umzuwandeln, die keine Phenylalaningruppe aufweisen, und daher den toxischen Effekt der Ochratoxine nicht mehr zeigen. Diese Metabolisierung der Ochratoxine geschieht durch ein der Carboxypeptidase A ähnliches Enzym, welches direkt die Amidbindung des Ochratoxins spaltet, oder auch durch einen Multienzymkomplex, durch den der Ring des Phenylalanins hydroxyliert wird und in weiterer Folge gespalten und abgebaut wird. Schließlich wird das verbliebene Aspartam abgespalten, wodurch wieder ein ungiftiger Metabolit der Ochratoxine erhalten wird. Dieser Weg kann in einfacher Weise wie folgt dargestellt werden:

Mit den erfindungsgemäßen Mikroorganismen, gewählt aus Bakterien und/oder Hefen, gelingt neben der Detoxifizierung von Ochratoxin A und Ochratoxin B auch jene der Metaboliten 4R-Hydroxyochratoxin A, 4S-Hydroxyochratoxin A, Ochratoxin C, Ochratoxin A Methylester, Ochratoxin B Methylester und Ochratoxin B Ethylester. Weiters gelingt mit diesen Mikroorganismen der Abbau und somit die Entgiftung von Zearalenonen.

Derartige Mikroorganismen können auch unbedenklich in Nahrungs- und Futtermitteln zum Einsatz gelangen, was bei einer Vielzahl von anderen Mykotoxine-spaltenden bzw. -abbauenden Bakterien und Hefen nicht unbedingt der Fall ist.

Die Tatsache, daß gemäß der vorliegenden Erfindung als Mikroorganismen sowohl aerobe als auch anaerobe, detoxifizierende Bakterien oder Hefen eingesetzt werden können, ist insbesondere von Bedeutung, da bei Aufnahme von mit entsprechenden Mikroorganismen kontaminierten Nahrungs- und/oder Futtermitteln auch eine Detoxifizierung nach der Aufnahme des Nahrungs- und/oder Futtermittels erreicht werden kann. Diese Detoxifizierung ist hiebei in jeder Stufe bzw. in jedem Abschnitt der Passage des Nahrungs- bzw. Futtermittels im Magen-Darm-Trakt möglich, da jedesmal gezielt die entsprechenden Mikroorganismen oder deren Kombinationen zum Einsatz gebracht werden können. Bekanntermaßen sind die Bedingungen im Magen-Darm-Trakt vom Magen bis zum Dickdarm ansteigend anaerob, was bedeutet, daß das Redoxpotential immer mehr abnimmt, so daß nach Aufnahme von mit entsprechenden Mykotoxinen bzw. Ochratoxinen und/oder Zearalenonen kontaminierten Nahrungs- und/oder Futtermitteln die Detoxifizierung zuerst mit aeroben Bakterien und/oder Hefen begonnen werden kann und am Ende eines Verdauungsdurchgangs bzw., sofern sich das Nahrungs- bzw. Futtermittel bereits in einem Darmabschnitt befindet, in welchem anaerobe Bedingungen vorherrschen, die Detoxifizierung mit entsprechenden anaeroben Bakterien und/oder Hefen durchgeführt werden kann.

Unter diesen weiteren, ebenfalls zum Abbau von Mikroorganismen befähigten Bakterien oder Hefen sind die im Folgenden genannten erfolgreich einsetzbar, wobei diese entweder in einem Medium oder Puffer zum Einsatz gelangen können oder auch in beiden Substanzen Wirkung zeigen.

| Stamm | Herkunft | Abbau in | |
|---|---|---|---|
| | | Medium | Puffer |
| Pseudomonas cepacia | Boden | 60 % | 100 % |
| Ochrobactrum | Boden/Wasser | 100 % | 100 % |
| Achromobacter | Boden/Wasser | 50 % | 100 % |
| Ralstonia | Boden/Wasser | 100 % | 100 % |
| Stenotrophomonas | Boden/Wasser | 100 % | 100 % |
| Rhodococcus erythropolis | DSM 1069 | 75 % | 90 % |
| Agrobacterium sp. | DSM 30201 | 20 - 100 % | 60 - 100 % |
| Agrobacterium tumefaciens | DSM 9674 | 25 - 40 % | 0 - 60 % |
| Pseudomonas putida | ATCC 700007 | 10 - 50 % | 0 % |
| Comomonas acidovorans | ATCC 11299a | 20 - 57 % | 0 - 50 % |
| Ascomyceten-Hefe | HA 168 | 95 % | 40 % |
| Cryptococcus flavus | HB 402 | 90 % | 100 % |
| Rhodotorula mucilaginosa | HB 403 | 20 % | 0 % |
| Cryptococcus laurentii | HB 404 | 50 % | 0 % |
| unbekannt | HB 508 | 30 % | 30 % |
| Trichosporon spec. nov. | HB 704 | 40 % | 40 % |
| unbekannt | HB 529 | 100 % | 95 % |
| Asocomyceten-Hefe | HA 1265 | 90 % | 0 % |
| Asocomyceten-Hefe | HA 1322 | 0 % | 95 % |
| Trichosporon ovoides | HB 519 | 100 % | 90 % |
| Triosporon dulcitum | HB 523 | 100 % | 100 % |
| Rhodotorula fujisanensis | HB 711 | 30 % | 0 % |
| Cryptococcus curvatus | HB 782 | 20 % | 95 % |
| Trichosporon guehoae | HB 892 | 50 % | 20 % |
| Trichosp. coremiiforme | HB 896 | 40 % | 20 % |
| Trichosporon mucoides | HB 900 | 100 % | 100 % |
| Trichosporon cutaneum | ATCC 46446 | 0 % | 70 % |
| Trichosporon dulcitum | ATCC 90777 | 0 % | 100 % |
| Trichosporon laibachii | ATCC 90778 | 0 % | 100 % |
| Trichosporon moniliiforme | ATCC 90779 | 0 % | 60 % |
| Cryptococcus humicolus | ATCC 90770 | 0 % | 30 % |
| Eubacterium sp. | F6 | 30 - 70 % | 100 % |
| Eubacterium callanderi | Di1_8 | 90 % | 100 % |
| Streptococcus sp. | Dü2_20 | | 40 - 70 % |
| Lactobacillus vitulinus | Ru8 | | 0 - 100 % |
| Stenotrophomonas nitritreducens | DSM 17575 | 100 % | 100 % |
| Stenotrophomonas nitritreducens | DSM 17576 | 50 % | 100 % |
| Stenotrophomonas sp. | DSM 13117 | 50 % | 95 % |

Als besonders vorteilhaft hat sich erwiesen, wie dies auch einer bevorzugten Weiterbildung der Erfindung entspricht, daß Bakterien und/oder Hefen insbesondere durch Lyophilisieren, Sprühtrocknen oder Mikroverkapseln stabilisiert sind. Durch die Stabilisierung der Mikroorganismen wird ihre Lebensfähigkeit und Lebensdauer verbessert bzw. erhöht, und überdies sind sie im stabilisierten Zustand universeller einsetzbar und somit in jeder beliebigen Anwendung zu jeder Zeit einsetzbar. Das Stabilisieren durch Lyophilisieren, Sprühtrocknen oder Mikroverkapseln ist an sich bekannt, wobei dies einfache und rasch durchführbare Verfahren sind, welche stabile, lebensfähige Mikroorganismen ergeben.

Gemäß einer Weiterbildung der Erfindung sind die Bakterien oder Hefen als zellfreier Extrakt oder Rohextrakt eingesetzt. Hiebei wird gemäß einer Weiterbildung bei Verwendung eines zellfreien Extrakts dieser in einer Lösung, insbesondere einer wäßrigen Lösung, eingesetzt. Wäßrige Lösungen eines zellfreien Extrakts haben den Vorteil, daß sie durch Aufsprühen auf die zu behandelnden Nahrungs- bzw. Futtermittel direkt auf der Oberfläche mit den kontaminierenden Mykotoxinen in Kontakt gelangen und eine Detoxifizierung bereits unmittelbar nach dem Aufsprühen des Extrakts erzielt werden kann. Die Verwendung eines Rohextrakts aus Bakterien und Hefen, der mittels Ultraschall, einem enzymatischen Aufschluß, durch eine Kombination aus Schockfrieren und Auftauen, Durchflußhomogenisator, French-Press, Autolyse bei hoher NaCl-Konzentration und/oder Kugelmühle erhalten ist, weist den Vorteil auf, daß ein derartiger Rohextrakt besonders schnell und unproblematisch erhalten werden kann, so daß insbesondere in Fällen, wo ein rascher Einsatz von detoxifizierenden bzw. Mykotoxine-abbauenden Substanzen erforderlich ist, die Verwendung eines Rohextrakts rasche und zuverlässige Ergebnisse bietet. Darüber hinaus können Rohextrakte unmittelbar in der Futtermittelherstellung eingesetzt werden, so daß die mit Mikroorganismen versetzten Futtermittel von den Tieren aufgenommen werden und die Mikroorganismen, insbesondere Hefen oder Bakterien, erst im Verdauungstrakt des Tiers ihre Wirkung entfalten. In diesem Zusammenhang kann erfindungsgemäß auch ein Gemisch, enthaltend Rohextrakt verschiedener detoxifizierender Bakterien und/oder Hefen, zum Einsatz gelangen.

Der Einsatz der Mikroorganismen in ungepufferter oder gepufferter Lösung, enthaltend Phosphat- oder Trishydrochloridpuffer mit pH-Werten zwischen 1 und 12, insbesondere zwischen 2 und 8, wie dies einer bevorzugten Ausführung entspricht, bietet den Vorteil, daß die Mikroorganismen unmittelbar mit Nahrungs- bzw. Futtermitteln verabreicht werden können und sie ihre Wirkung im Magen-Darm-Trakt an den Bereichen, wo das Redoxpotential für eine optimale Wirkung der eingesetzten Mikroorganismen geeignet ist, entfalten. Durch Einsatz einer gepufferten Lösung gelingt eine unmittelbare Anpassung an die jeweiligen pH-Werte im Magen-Darm-Trakt, so daß durch die mit der gepufferten Lösung an Mikroorganismen versetzten Nahrungs- bzw. Genußmittel keine Verschiebung bzw. Störung des im Magen-Darm-Trakts vorherrschenden pH-Werts bewirken, was einerseits die leichte Verdaulichkeit der versetzten Nahrungs- bzw. Futtermittel erhöht und andererseits zu keiner Störung der Verdauung in dem Magen-Darm-Milieu führt.

Eine weitere Aufgabe der vorliegenden Erfindung ist es, Substanzen bzw. Mikroorganismen zur Verfügung zu stellen, welche zur Detoxifizierung von Nahrungs- oder Genußmitteln eingesetzt werden können, ohne daß sie für die sie aufnehmenden Lebewesen eine Schädigung bzw. Beeinträchtigung mit sich bringen und ohne daß die mit ihnen behandelten Nahrungs- bzw. Genußmittel neben einer Detoxifizierung der Mykotoxine, die auf den Nahrungs- bzw. Genußmitteln vorhanden waren, irgendwelchen Beeinträchtigungen bzw. Veränderungen unterliegen.

Zur Lösung dieser Aufgaben wurde erfindungsgemäß gefunden, daß die Verwendung von Bakterien, gewählt aus Sphingomonas sp. DSM 14170 und DSM 14167, Stenotrophomonas nitritreducens DSM 14168, Stenotrophomonas sp. DSM 14169, Ralstonia eutropha DSM 14171 und Eubacterium sp. DSM 14197, und/oder Hefen, gewählt aus Trichosporon spec. nov. DSM 14153, Cryptococcus sp. DSM 14154, Rhodotorula yarrowii DSM 14155, Trichosporon mucoides DSM 14156 und Trichosporon dulcitum DSM 14162, es ermöglicht, die auf der Oberfläche von Nahrungs- oder Futtermitteln vorhandenen Mykotoxine, nämlich Ochratoxine, durch Abspalten der Phenylalaningruppe zu detoxifizieren bzw. Zearalenone abspalten, ohne die mit ihnen behandelten Nahrungs- bzw. Futtermittel in irgendeiner Weise zu beeinträchtigen bzw. zu beeinflussen.

Als insbesondere geeignet für diesen Zweck hat sich die Verwendung von Trichosporon spec. nov. DSM 14153, Eubacterium sp. DSM 14197 oder Stenotrophomonas nitritreducens DSM 14168 erwiesen, mit welchen ein insbesondere vollständiger Abbau von Mykotoxinen, nämlich Ochratoxinen und/oder Zearalenonen, gelingt und gegen welche Mikroorganismen keinerlei Bedenken bestehen.

Um eine insbesondere wirtschaftliche Detoxifi.zierung von Mykotoxinen, insbesondere in Nahrungs- oder Futtermitteln, zu erreichen, werden Mischkulturen oder Kombinationen von mehreren Bakterien und/oder Hefen zur Detoxifizierung von Ochratoxinen und/oder Zearalenonen in Nahrungsmitteln und/oder Futtermitteln verwendet.

Eine weitere Aufgabe der vorliegenden Erfindung ist es, ein Verfahren zur biologischen Entgiftung von Mykotoxinen, nämlich Ochratoxinen und/oder Zearalenonen, in Nahrungs- und Futtermitteln mit einem Mikroorganismus zur Verfügung zu stellen, mit welchem es gelingt, die kontaminierenden Toxine vollständig und rasch direkt nach Kontaktierung mit Nahrungs- oder Futtermittel oder im Verdauungstrakt der diese Nahrungs- oder Futtermittel aufnehmenden Lebewesen zu dekontaminieren.

Zur Lösung dieser Aufgaben ist das erfindungsgemäße Verfahren im wesentlichen dadurch gekennzeichnet, daß ein erfindungsgemäßer Mikroorganismus, insbesondere erfindungsgemäße Bakterien und/oder Hefen, mit dem Nahrungs- oder Futtermittel in einer Menge von 0,01 Gew.-% bis 1 Gew.-%, insbesondere 0,05 Gew.-% bis 0,5 Gew.-%, vermischt wird. Indem das Nahrungs- oder Futtermittel mit dem erfindungsgemäßen Mikroorganismus, insbesondere den erfindungsgemäßen Bakterien oder Hefen, in fester Form vermischt wird, kann eine stabile Form von Nahrungs- bzw. Futtermitteln, die mit dem entsprechend stabilisierten Mikroorganismus versetzt sind, erzielt werden. Wenn ein derartiges mit dem erfindungsgemäßen Mikroorganismus versetztes Nahrungs- oder Futtermittel aufgenommen wird, kommt es im Zuge der Einspeichelung zur Ausbildung einer Suspension und die Entgiftung des Nahrungs- bzw. Futtermittels, insbesondere der Abbau der Ochratoxine, beginnt unmittelbar nach Aufnahme des Nahrungs- bzw. Futtermittels durch den Menschen bzw. das Tier. Auf diese Weise gelingt ein vollständiger Abbau von schädlichen Mykotoxinen, nämlich Ochratoxinen und/oder Zearalenonen, im Magen-Darm-Trakt des aufnehmenden Wirtstiers, so daß der Organismus durch schädliche Mykotoxine in keiner Weise belastet wird.

Wenn angestrebt wird, daß bereits entgiftete Nahrungs- bzw. Futtermittel aufgenommen werden, wird gemäß einer Weiterbildung der Erfindung das Vermischen der Nahrungs- bzw. Futtermittel durch Rühren in einer wäßrigen Suspension des Mikroorganismus, enthaltend 20 bis 99 Gew.-%, insbesondere 35 Gew.-% bis 85 Gew.-% Wasser bei Temperaturen von 10 bis 60 °C, insbesondere 15 bis 45 °C, für 2 min bis 12 h, insbesondere 5 min bis 2 h, durchgeführt. Dadurch, daß die Nahrungs- oder Futtermittel durch Rühren in einer wäßrigen Suspension des Mikroorganismus behandelt werden, gelingt einerseits ein inniger Kontakt der zu behandelnden Nahrungs- oder Futtermittel mit den detoxifizierenden Mikroorganismen und andererseits eine schonende Behandlung der Mikroorganismen, wodurch sichergestellt wird, daß diese durch die Vermischung mit dem Nahrungs- oder Futtermittel nicht verschlechtert bzw. abgetötet werden. Bei diesem Vermischen ist es insbesondere wichtig, zu beachten, daß sowohl die Temperaturen des Vermischens nicht zu hoch bzw. zu tief werden, als auch daß die Dauer und Zusammensetzung der Aufschlämmung bzw. Suspension vollständig in Übereinstimmung mit der vorliegenden Erfindung eingehalten werden, um eine Zerstörung bzw. Abtötung der Mikroorganismen mit Sicherheit hintanzustellen.

Die Erfindung wird nachfolgend anhand von Beispielen betreffend die Isolierung der Mikroorganismen und ihrer Wirkungsweise näher erläutert.

### 1. Anzucht, Produktion und Gewinnung der Hefe Trichosporum spec. nov. (DSM 14153)

Für die Aufzucht der Hefe wird die folgende Kulturlösung verwendet:
10 g Hefeextrakt
20 g Malzextrakt
10 g Glucose
5 g Pepton
400 ppm Ochratoxin A
1 l RO-Wasser
pH-Wert 5,5

Diese wird für 25 min bei 121 °C im Autoklaven behandelt. In einem 100 ml Erlenmeyerkolben werden 30 ml einer Vorkultur angelegt (Inokulumsrate 0,33 %). Die Inkubation erfolgt für 72 h bei 25 °C am Schüttler. Die erreichte Keimzahl beträgt ca. 5 x 10⁷/ml.

Danach dienen diese 30 ml der Vorkultur als Inokulum für eine Fermentation in einem 75 l Fermenter. Für diese Fermentation wird das folgende Kultivierungsmedium eingesetzt:

| | |
|---|---|
| Malzextrakt | 4 g/l |
| Hefeextrakt | 10 g/l |
| Pepton | 5 g/l |
| Glucose | 10 g/l |
| Antischaummittel | 0,1 % |
| pH-Wert | 5,5 |

Als weitere Parameter werden ein pO₂ von 40 % und eine maximale Belüftungsrate von 3 m³/h eingestellt. Der pH-Wert zu Beginn beträgt 5,00, ändert sich jedoch im Laufe des Wachstumsprozesses (er steigt an bis auf 8,5). Nach ca. 40 - 44 h kann der Inhalt als Inokulum für den 3,6 m³ Produktionsfermenter herangezogen werden. Für diesen wird das folgende Medium verwendet:

| | |
|---|---|
| Malzextrakt | 17 g/l |
| Hefeextrakt | 5 g/l |
| Pepton | 2 g/l |
| Antischaummittel | 0,1 % |

Die Belüftungsrate beträgt 15 m³ Luft pro Stunde. Nach 40 - 48 h können die Zellen mittels Durchflußseparator aufkonzentriert werden. Mittels Separator kann die Fermentationsbrühe ca. 1:10 aufkonzentriert werden, wobei eine Keimzahl von ca. 3 x 10⁹/ml erhalten wird.

Die anschließende Stabilisierung geschieht mittels Gefriertrocknung oder durch Sprühtrocknung. Als Kryoprotektor für die Gefriertrocknung dient Molkepulver.

Es werden 10 % bezogen auf das Konzentratvolumen zugesetzt. Anschließend wird das Konzentrat bei -80 °C gefroren. Die Gefriertrocknung geschieht bei einem Druck von 0,400 mbar, bei einer Stellflächentemperatur von 20 °C. Die Dauer beträgt bei einer Schichtdicke von 1,5 cm ca. 30 h.

Die Parameter für die Sprühtrocknung:
- Eintrittstemperatur des Trocknungsmediums (Luft): 160 °C
- Austrittstemperatur: 80 °C
- Druck: 3 bar

### 2. Anzucht, Produktion und Gewinnung des Bakteriums Stenotrophomonas nitritreducens (DSM 14168)

Die Anzucht des Bakteriums geschieht in Nährbouillon, einmal in Oxoid CM 001 B und das zweite Mal in CM 067 B mit 400 ppb Ochratoxin A. 30 ml.des Mediums werden in einem 100 ml Erlenmeyerkolben 25 min lang bei 121 °C autoklaviert. Als Inokulum dient 1,5 ml eines Röhrchens aus der "Working Cell Bank". Die Inkubation erfolgt für 72 h bei 30°C am Schüttler.

Danach dienen diese 30 ml der Vorkultur als Inokulum für eine Fermentation in einem 75 1 Fermenter. Für diese Fermentation wird das folgende Kultivierungsmedium eingesetzt:

| | |
|---|---|
| Pepton aus Fleisch | 5 g/l |
| Fleischextrakt | 3 g/l |
| Antischaummittel | 0,1 % |

Als weitere Parameter werden ein pO₂ von 40 % und eine maximale Begasungsrate von 3 m³/h eingestellt. Die Rührergeschwindigkeit beträgt 200 U/min. Der pH-Wert zu Beginn beträgt ca. 6,8 bis 6,9, ändert sich jedoch im Laufe des Wachstumsprozesses, er steigt an bis auf 8,3. Nach ca. 40 - 44 h kann der Inhalt als Inokulum für den 3,6 m³ Produktionsfermenter herangezogen werden. Für diesen wurde das folgende Medium verwendet:

| | |
|---|---|
| Sojabohnenmehl | 17 g/l |
| Hefeextrakt | 5 g/l |
| Pepton | 2 g/l |
| Antischaummittel | 0,1 % |

Die Belüftungsrate ist auf 15 m³ Luft pro Stunde eingestellt. Die Rührergeschwindigkeit beträgt 250 U/min.

Nach 40 - 48 h können die Zellen mittels Durchflußseparator aufkonzentriert werden. Das Aufkonzentrierungsverhältnis beträgt ungefähr 1:100.

Die anschließende Stabilisierung geschieht mittels Gefriertrocknung oder durch Sprühtrocknung. Als Kryoprotektor für die Gefriertrocknung dient Molkenpulver.

Zumeist werden 10 % bezogen auf das Konzentratvolumen zugesetzt. Anschließend wird das Konzentrat bei -80 °C (10h) oder mit flüssigem Stickstoff (2 h) gefroren. Die Gefriertrocknung erfolgt bei einem Druck von 0,400 mbar, bei einer Stellflächentemperatur von 20 °C. Die Dauer beträgt bei einer Schichtdicke von 1,5 cm ca. 30 h.

Die Parameter für die Sprühtrocknung:
- Eintrittstemperatur des Trocknungsmediums (Luft): 160 °C
- Austrittstemperatur: 80 °C
- Druck: 3 bar

### 3. Anzucht, Produktion und Gewinnung des Bakteriums Eubacterium sp. (DSM 14197)

Zur Anzucht dieses anaeroben Bakteriums wird das folgende Medium herangezogen:

| | |
|---|---|
| D(+)-Glucose | 4 g/l |
| Pepton aus Casein | 2 g/l |
| Hefeextrakt | 2 g/l |
| Minerallösung I | 75 ml/l |
| [KH₂PO₄ 6 g/l] | |
| Minerallösung II [K₂HPO₄ 6 g/l; (NH₄)SO₄ 6 g/l; NaCl 12 g/l; MgSO₄x7H₂O 2,5 g/l; CaCl₂ x 2 H₂O 3g/l] | 75 ml/l |
| Hämin | 1 mg/l |
| Fettsäurenmischung | 3,1 ml/l |
| Cystein-HCl | 0,5 g/l |
| Resazurin | 1 mg/l |
| Ochratoxin A | 400 ppb |
| pH | 6,9 |

Die Anzucht erfolgt in einem 100 ml Pyrexfläschchen mit Silikonseptum. 80 ml des autoklavierten Mediums werden abgefüllt und mit einem KH₂PO₄/Na₂HPO₄-Puffer (pH 7) versetzt. Nach dem Zusatz des Inhaltes eines Cryovials der Working Cell Bank wird der Kopfraum des Gefäßes mit N₂ begast (1 min). Nach dem Verschließen des Fläschchens wird dieses 72 h lang bei 37 °C inkubiert.

Danach werden 4,5 l der obengenannten Kulturlösung in einer 5 l Schottflasche autoklaviert. Diese besitzt einen Entnahmestutzen und 2 Stutzen mit Sterilfilter (zum Begasen des Inokulums). Nach dem Abkühlen des Mediums auf 37°C werden zuerst die Pufferlösung (1 % des Phosphatpuffers) und anschließend die 80 ml Inokulum hinzugefügt. Nach der Begasung des Kopfraumes mit Stickstoff (5 min lang) werden die Öffnungen mit Schlauchklemmen verschlossen und das Inokulum bei 37 °C ca. 64 h lang bebrütet. Nach der Reinheitskontrolle kann dieses als Inokulum für einen 1 m³ Fermenter (Füllvolumen 700 l) verwendet werden.

Zur Produktion wird das folgende Medium eingesetzt:

| | |
|---|---|
| Glucose | 10 g/l |
| Hefeextrakt | 5 g/l |
| Pepton | 2 g/l |
| Cystein-HCl | 0,5 g/l |
| pH-Wert | 7,00 |

Nach der Sterilisation des Mediums im Fermentationstank (40 min, 121 °C , 1,21 bar) und der Rückkühlung auf 37 °C wird das Inokulum hinzugefügt. Der Kopfraum des Fermenters wird mit N₂ gespült. Die Rührergeschwindigkeit beträgt 100 U/min, zur pH-Wert-Regulierung wird Natronlauge (8 Mol/l) verwendet. Das Redoxpotential liegt zu Beginn bei ca. -240 mV und nimmt im Laufe des Wachstums auf über - 500 mV ab. Die Fermentationszeit beträgt ca. 48 h. Die Aufkonzentrierung geschieht mittels Durchflußseparator.

Die anschließende Stabilisierung geschieht mittels Gefriertrocknung, Mikroverkapselung oder durch Sprühtrocknung. Als Kryoprotektor für die Gefriertrocknung dient Molkepulver.

Es werden 10 % bezogen auf das Konzentratvolumen zugesetzt. Anschließend wird das Konzentrat bei -80 °C (10 h) oder mit flüssigem Stickstoff (2 h) gefroren. Die Gefriertrocknung geschieht bei einem Druck von 0,400 mbar, bei einer Stellflächentemperatur von 20 °C. Die Dauer beträgt bei einer Schichtdicke von 1,5 cm ca. 30 h.

Der Schutz des Mikroorganismus während der Lagerung bzw. vor ungünstigen Lebensbedingungen geschieht durch Wirbelschichtgranulation mit einem pflanzlichen Fett (Holtmelt Verfahren, Top Spray).

| | |
|---|---|
| Sprührate: | ca. 80 - 150 g/min |
| Zulufttemperatur: | 50 °C |
| Sprühdruck: | 3 bar |
| Luftmenge: | 750 - 1500 m³/h |
| Produkttemperatur: | < 47 °C |

Die Parameter für die Sprühtrocknung:
- Eintrittstemperatur des Trocknungsmediums (Inertgas): 160 °C
- Austrittstemperatur: 80 °C
- Druck: 3 bar

### 4. Entgiftung von Ochratoxin A (OTA) durch Bakterien- und Hefeprodukte gemäß den Beispielen 1 bis 3

Von den in Beispiel 1 bis 3 erhaltenen Produkten wird eine logarithmische Verdünnungsreihe bis zur Stufe 10⁻⁴ in physiologischer Kochsalzlösung angefertigt. Von den Stufen 10⁻¹ bis 10⁻⁴ werden je 2 ml in 18 ml des entsprechenden Mediums (Minimalmedium (Na₂HPO₄ 2,44 g/l; KH₂PO₄ 1,52 g/l; (NH₃)₂SO₄ 0,50 g/l; MgSO₄ x 7H₂O 0,20 g/l, CaCl2 x 2H₂O 0,05 g/l), Hefemedium oder Nährbouillon (Oxoid CM001B)), welche mit 200 ppb OTA versetzt sindr, pipettiert. Die verwendeten Kolben werden unter den entsprechenden Bedingungen am Horizontalschüttler inkubiert. Nach 2,5, 5 und 24 h werden Proben entnommen und mittels Hochleistungschromatographie auf OTA-Spaltung untersucht. In der Verdünnungsstufe 10⁻³ (entspricht Produktkeimzahlen von 10⁵) haben die Hefen im Minimalmedium nach 5 h 90 % und 24 h 100 % des Ochratoxin A gespalten.

Wird als Testmatrix das komplexe Hefemedium herangezogen, so zeigen die Produkte nach 6 h in der Verdünnungsstufe 10⁻² eine Spaltungsrate von 90 %. Nach 24 h ist das gesamte OTA entgiftet.

Die Bakterienprodukte haben im Minimalmedium in der Verdünnungsstufe 10⁻³ (Keimzahl von 10⁶ - 10⁹) nach 2,5 h 40 bis 100 % und nach 24 h 100 % des Ochratoxin A entgiftet. In Nährbouillon verläuft die Entgiftung etwas langsamer - nach 2,5 h sind in der Stufe 3 40 bis 50 % und 24 h 80 bis 100 % detoxifiziert. Diese Versuche zeigten, daß die Mikroorganismen in stabile Produkte überführt werden können, die sowohl in einem minimalen als auch in einem komplexen Medium eine Detoxifikationsaktivität aufweisen.

### 5. Ochratoxinabbau (OTA) durch Lyophilisate in stimuliertem Darmmilieu

### Testmodell A

Mit diesem Modell werden Lyophilisate der Hefestämme DSM 14153, DSM 14154, DSM 15155, DSM 14156 und DSM 14162 sowie der aeroben (DSM 14170, DSM 14167, DSM 14168 und DSM 14169) und anaeroben (DSM 14197) Bakterienstämme untersucht. Schlachtfrischer Schweinedünndarm wird in ca. 15 cm lange Stücke geschnitten, die dann zu jeweils 100 ml OTA [200 ppb]-hältigem Minimalmedium gegeben wurden. Die Ansätze wurden.schließlich direkt mit 1 g Lyophilisat inokuliert und bei 35 °C inkubiert. Nach 0, 6, 24 und 48 h wurden Proben für eine nachfolgende OTA-Analyse mittels HPLC gezogen und tiefgefroren (-20 °C) bis zur Analyse aufbewahrt.

Unter den Hefen erwiesen sich die Keime DSM 14153, DSM 14156 und DSM 14162 als am höchsten aktiv. Bereits nach den ersten 6 Inkubationsstunden waren 70 - 90 %, 50 - 90 % bzw. 80 - 90 % des vorhandenen Toxins transformiert (nach 24 h: 90 - 95 %). Die beiden übrigen getesteten Hefen (DSM 14154, DSM 14155) hinkten bezüglich Aktivität hinter den genannten drei Stämmen her (0 - 20 % Abbau nach 6 h; 30 - 50 % nach 24 h; 80 % nach 48 h).

Bei den aeroben Bakterien war der Keim DSM 14168 am besten; nach 6 h waren bereits 50 - 100 % des vorhandenen Toxins umgesetzt, nach 24 h 80 - 100 %. Auch DSM 14169 erwies sich als durchaus "akzeptabel": nach 6 h waren 0 - 90 % OTA detoxifiziert, nach 24 h 70 - 95 %. Die beiden übrigen Keime waren deutlich leistungsschwächer (0 - 40 % nach 6 ; 50 - 60 % nach 24 h; 60 - 80 % nach 48 h).

Das anaerobe Dünndarmisolat DSM 14197 degradierte das vorhandene Mykotoxin nach 6 Inkubationsstunden in einem Ausmaß von 0 bis 60 %; nach 24 h waren zwischen 50 und 100 % OTA umgesetzt.

Analoge Untersuchungen wurden mit folgenden Keimen durchgeführt:

| | |
|---|---|
| Dünndarmisolat F6: | 90 - 95 % nach 24 h |
| Dickdarmisolat Di 1-8: | 80 - 95 % nach 24 h |
| Trichosporon ovoides: | 40 - 50 % nach 24 h |
| Triosporon dulcitum: | 50 - 90 % nach 24 h |
| Cryptococcus curvatus: | 40 - 50 % nach 24 h |
| Trichosporon laibachii: | 50 % nach 24 h |
| Stenotrophomonas nitritreducens: | 60 - 95 % nach 24 h |
| Stenotrophomonas sp.: | 50 - 70 % nach 24 h |

Dieses Modell zeigte, daß OTA in einem Puffermilieu, das einen Darmabschnitt mit dem entsprechenden Milieu (Nährstoffe und die Darmflora) enthält, durch die hergestellten Produkte deaktiviert werden kann.

### Testmodell B

Mit diesem Modell wurden Lyophilisate der Hefestämme DSM 14153, DSM 14156 und DSM 14162 sowie der Bakterienstämme DSM 14168 (aerob), DSM 14169 (aerob) und DSM 14197 (anaerob) untersucht.

Schlachtfrischer Schweinedünndarm wurde in ca. 25 cm lange Stücke geschnitten, die mittels Bindfaden an ihren Enden zugebunden wurden. 1 g des zu untersuchenden Produkts wurde in ein 50 ml Zentrifugenröhrchen eingewogen und in 20 ml OTA [200 ppb]-hältigem Testmedium (aerobe Keime und Hefen → Minimalmedium; anaerobe Keime → Anaerobpuffer) resuspendiert. Von der gut gemischten Suspension ausgehend wurden optional auch Zehnfachverdünnungen hergestellt. Die gemischte(n) Suspension(en) wurden dann jeweils direkt in ein Darmstück injiziert. Nach dem Ziehen der Nullprobe direkt aus dem Darmstück wurde dieses in einer 250 ml Pyrexflasche hängend (d.h. der Bindfaden eines Endes wurde mit dem Schraubverschluß der Flasche befestigt) bei 35 °C inkubiert. Nach 6, 24 und 48 h wurden weitere Proben für eine nachfolgende OTA-Transformationsanalyse mittels HPLC gezogen.

Im Falle der Hefen (ca. 10⁷ KBE/ml) wurde nach 6 h ein OTA Abbau von bis zu 90 % (DSM 14153) registriert. Nach spätestens 24 h konnten dann für alle Produkte vergleichbar hohe Aktivitäten nachgewiesen werden (80 - 100 %).

Auch mit den zehn- und hundertfachen Verdünnungen der Lyophilisate wurden vergleichbare Ergebnisse erzielt. Mit den beiden aeroben Bakterien DSM 14168 und DSM 14169 wurden ähnliche Resultate erhalten. Nach 6 h waren 20 - 60 % des OTA transformiert, nach 24 h 80 - 95 %. Der anaerobe Keim DSM 14197 wies nach 6 h eine Abbauleistung zwischen 40 und 50 % auf, die nach 24 h auf 90 % gesteigert werden konnte. Darmabschnitte, die mit OTA, aber ohne Produkte inkubiert wurden, zeigten keine Entgiftungsaktivität.

Mit diesen Versuchen wird bewiesen, daß die Ochratoxindetoxifizierenden Mikroorganismen dieses Toxin auch in einem einem Darm entsprechenden Milieu abbauen können. Dadurch ist insbesondere die Verwendung der Mikroorganismen als Nahrungs- bzw. Futtermittelzusatz zur Detoxifizierung von Ochratoxinen eindeutig nachgewiesen.

### 6. Detoxifizierung von Lebens- und Futtermitteln

Die Ochratoxin entgiftenden Mikroorganismen wurden entsprechend den Beispielen 1 bis 3 unter den entsprechenden Bedingungen ca. 66 h lang angezüchtet. Je 25 ml der Suspensionen wurden für 15 min bei 3210 x g abzentrifugiert und in einem adäquaten Volumen des Minimalmediums, welches mit 200 ppb OTA versetzt war, aufgenommen. Die entstehende Suspension wurde zur Inokulation von 25 g bzw. 25 ml Lebensmittel, Kaffeepulver, Maisgrieß, Weizengrieß, Bier und Wein, herangezogen. Nach sorgfältiger Vermengung des Lebensmittels mit der Mikroorganismensuspension wurde eine Probe (= Nullprobe) gezogen. Die Inkubation der Ansätze erfolgte für 9 Tage bei 25 °C. Danach wurden 5 g der Probe im Vergleich zur Nullprobe analysiert. Zusätzlich wurden Blindwerte mitinkubiert. Diese wurden mit OTA, jedoch ohne Mikroorganismen versehen. Zur Analyse des Ochratoxin in den flüssigen Lebensmitteln wurde genau 1 ml des von den Mikroorganismen befreiten Lebensmittels mit 0,5 ml 1M Phosphorsäure angesäuert und mit 5 ml Methylenchlorid extrahiert. 5 ml des Extraktes wurden unter Stickstoff eingetrocknet. Jede Probe wurde zweimal aufgearbeitet, der Rückstand nach dem Trocknen wurde einmal in Acetonitril/Wasser/Essigsäure (45:54:1) und einmal in Toluol/Essigsäure (99:1) aufgenommen. Die Analysen der Proben erfolgten sowohl mittels HPLC als auch mittels TLC. Bei der Analyse des Weizengrießes wurden 5 g der Probe in eine 100 ml Schottflasche eingewogen und mit 20 ml Acetonitril/Wasser (60:40) bei 170 U/min eine Stunde lang geschüttelt. Nach dem Abfiltrieren wurde dieser Extrakt direkt mittels HPLC analysiert. Die Aufarbeitung für die OTA-Analysen aus Maisgrieß und Kaffee war etwas aufwendiger. Hier wurden 5 g der Probe in eine 100 ml Schottflasche eingewogen und mit 20 ml Acetonitril:Wasser (60:40) eine Stunde lang geschüttelt. Nach dem Abfiltrieren wurden 4 ml des Extrakts mit 44 ml PBS-Puffer (0,1 % Tween 20) versetzt und auf eine Immunoaffinitätssäule aufgegeben. Anschließend erfolgte die HPLC-Analyse. Es wurde sowohl die Abnahme des OTA als auch das Auftreten des Metaboliten OTα bestimmt. In den Kaffee- und Maisproben konnte aufgrund der eingesetzten Säulenaufreinigung kein OTα nachgewiesen werden. Folgende Abbauraten konnten erhalten werden:

| Stamm | OTA-Reduktion in Prozent | | | | |
|---|---|---|---|---|---|
| | Bier | Wein | Mais | Weizen | Kaffee |
| DSM 14153 | 100 (+) | 99 (+) | 94 (+) | 100 (+) | ~67 (+) |
| DSM 14154 | 100 (+) | 94 (+) | 50 (+) | 100 (+) | 0 (-) |
| DSM 14155 | 30 (+) | 0 (-) | 99 (+) | 100 (+) | 0? (-) |
| DSM 14156 | 100 (+) | 95 (+) | 96 (+) | 100 (+) | 30 (+) |
| DSM 14162 | 83 (+) | 12 (+) | 100 (+) | 100 (+) | 0 (-) |
| DSM 14170 | 75 (+) | 0 (-) | 0 (-) | 89 (+) | 0 (-) |
| DSM 14167 | 100 (+) | 4 (+) | 39 (+) | ~90 (+) | 0 (-) |
| DSM 14168 | 100 (+) | 0 (-) | 50 (+) | 94 (+) | 0 (-) |
| DSM 14169 | 100 (+) | 0 (-) | 0 (-) | 91 (+) | 0 (-) |
| DSM 14171 | 100 (+) | 0 (-) | 79 (+) | 81 (+) | 0 (-) |

### 7. Abbau von Mykotoxinen

Die Mikroorganismen wurden ca. 66 h lang angezüchtet. Danach wurden diese abzentrifugiert (3210 x g, 15 min, Raumtemperatur) und die erhaltenen Pellets im Minimalmedium resuspendiert Dem Minimalmedium wurden 1 ppm Deoxynivalenol, 1 ppm Fumonisin B₁, 1 ppm Zearalenon, 200 ppb Aflatoxin B₁ und 2 ppm Citrinin zugesetzt. Bevor die Ansätze bei 30 °C inkubiert wurden, erfolgte eine Probenahme ("Nullprobe"). Die Inkubationsdauer betrug 96 h. Die Ansätze wurden in Doppelbestimmung durchgeführt, wobei für die HPLC-Analyse einmal der Überstand (nach Zentrifugation) und einmal der gesamte Ansatz untersucht wurde. Zur Aufreinigung wurden 3 ml der Überstände bzw. 2 ml der gesamten Probe auf 15 g Extrelutmaterial gegeben. Nach 15 min wurden die Proben mit 40 ml Ethylacetat eluiert. Je 7 ml des Ethylacetats wurden eingetrocknet und im entsprechenden Laufmittel aufgenommen. Die Analyse von Aflatoxin B₁ und Fumonisin B₁ erfolgte nach vorangegangener Derivatisierung.

Die Proben nach 96 h wurden im Vergleich zu den Proben zu Beginn auf Abbau der entsprechenden Toxine untersucht. Dazu wurden bei DON, ZON und AFB₁ sowohl die Überstände (Abtrennung der Biomasse durch Zentrifugation) als auch die Gesamtprobe (mit Biomasse) analysiert. Die Ergebnisse sind in Tabelle 2 dargestellt.

**Tabelle 2**

| Stämme | ZON-Abbaurate [%] | | FB₁-Abbaurate [%] | AFB₁-Abbaurate [%] | | CIT-Abbaurate [%] |
|---|---|---|---|---|---|---|
| | Überstand | gesamt | Überstand | Überstand | gesamt | Überstand |
| DSM 14170 | 0 | 24 | 0 | 0 | 0 | 100 |
| DSM 14167 | 0 | 28 | 0 | 0 | 10 | 0 |
| DSM 14168 | 0 | 32 | 0 | 0 | 10 | 0 |
| DSM 14169 | 88 | 90 | 0 | 8 | 46 | 0 |
| DSM 14171 | 0 | 43 | 0 | 0 | 24 | 0 |
| DSM 14153 | 100 | 100 | 19 | 20 | 13 | 0 |
| DSM 14154 | 19 | 67 | 22 | 64 | 63 | 0 |
| DSM 14155 | 81 | 100 | 29 | 20 | 38 | 0 |
| DSM 14156 | 100 | 100 | 6 | 0 | 61 | 0 |
| DSM 14162 | 17 | 62 | 8 | 0 | 0 | 0 |

Aus diesen Versuchen zeigt sich deutlich, daß einige Mykotoxine, wie Zearalenon (ZON), Aflatoxin B₁ (AFB₁), Fumonisin B₁ (FB₁), mit den Mikroorganismen gemäß der vorliegenden Erfindung teilweise extrem gut abgebaut werden können. Citrinin (CIT) konnte lediglich mit dem Bakterium Sphingomonas sp. (DSM 14170) zu 100 % abgebaut werden.

Zusammenfassend läßt sich festhalten, daß mit den Mikroorganismen gemäß der vorliegenden Erfindung insbesondere ein Abbau von Ochratoxinen in Nahrungs- und Futtermitteln und auch im Darmmilieu sehr gut möglich ist, wobei jedoch auch ein Abbau von Zearalenon, Citrinin und dgl. teilweise gute Ergebnisse zeigte.
Anlage zu PCT-Anmeldung PCT/AT02/00356
Anmelder: Erber Aktiengesellschaft et al.

Liste gemäß Regel 13bis, Absatz 4 der Ausführungsordnung zum Vertrag über die internationale Zusammenarbeit auf dem Gebiet des Patentwesens

Sämtliche der in der vorliegenden PCT-Anmeldung PCT/AT02/00356 genannten Mikroorganismen wurden bei der DSMZ - Deutsche Sammlung von Mikroorganismen und Zellkulturen GmbH, Mascheroder Weg. 1b, 38123 Braunschweig, Deutschland (DE), hinterlegt.

| Eingangsnummer | Eingangsdatum |
|---|---|
| DSM 14156 | 08.03.2001 |
| DSM 14155 | 08.03.2001 |
| DSM 14154 | 08.03.2001 |
| DSM 14153 | 08.03.2001 |
| DSM 14197 | 15.03.2001 |
| DSM 14171 | 08.03.2001 |
| DSM 14169 | 08.03.2001 |
| DSM 14168 | 08.03.2001 |
| DSM 14167 | 08.03.2001 |
| DSM 14170 | 08.03.2001 |
| DSM 14162 | 08.03.2001 |

## Patentansprüche

1. Mikroorganismus zur biologischen Entgiftung von Mykotoxinen, nämlich Ochratoxinen und/oder Zearalenonen, **dadurch gekennzeichnet, daß** ein Mikroorganismus, insbesondere aerobe oder anaerobe, detoxifizierende Bakterien oder Hefen, eingesetzt ist bzw. sind, welche(s) die Phenylalaningruppe der Ochratoxine abspalten bzw. Zearalenone abbauen, wobei die Mikroorganismus detoxifizierenden Bakterien aus Sphingomonas sp. DSM 14170 und DSM 14167, Stenotrophomonas nitritreducens DSM 14168, Stenotrophomonas sp. DSM 14169, Ralstonia eutropha DSM 14171 und Eubacterium sp. DSM 14197 gewählt sind, und/oder die detoxifizierenden Hefen aus Trichosporon spec. nov. DSM 14153, Cryptococcus sp. DSM 14154, Rhodotorula yarrowii DSM 14155, Trichosporon mucoides DSM 14156 und Trichosporon dulcitum DSM 14162 gewählt sind.

2. Mikroorganismus nach Anspruch 1, **dadurch gekennzeichnet, daß** die Bakterien und/oder Hefen insbesondere durch Lyophilisieren, Sprühtrocknen oder Mikroverkapseln stabilisiert sind.

3. Mikroorganismus nach Anspruch 1 oder 2, **dadurch gekennzeichnet, daß** die Bakterien oder Hefen als zellfreier Extrakt oder Rohextrakt eingesetzt sind.

4. Mikroorganismus nach Anspruch 3, **dadurch gekennzeichnet, daß** der Mikroorganismus als zellfreier Extrakt in einer Lösung, insbesondere einer wäßrigen Lösung, eingesetzt ist.

5. Mikroorganismus nach Anspruch 3, **dadurch gekennzeichnet, daß** der Rohextrakt aus den Bakterien und Hefen mittels Ultraschall, einen enzymatischen Aufschluß, durch eine Kombination aus Schockfrieren und Auftauen, Durchflußhomogenisator, French-Press, Autolyse bei hoher NaCl-Konzentration und/oder Kugelmühle erhalten ist.

6. Mikroorganismus nach einem der Ansprüche 1 bis 4, **dadurch gekennzeichnet, daß** der Mikroorganismus in gepufferter Lösung, enthaltend Acetat-, Citrat-, Phosphat- oder Trishydrochloridpuffer mit pH-Werten zwischen 1 und 12, insbesondere zwischen 2 und 8, eingesetzt ist.

7. Verwendung von Bakterien, gewählt aus: Sphingomonas sp. DSM 14170 und DSM 14167, Stenotrophomonas nitritreducens DSM 14168, Stenotrophomonas sp. DSM 14169, Ralstonia eutropha DSM 14171 und Eubacterium sp. DSM 14197 und/oder Hefen, gewählt aus: Trichosporon spec. nov. DSM 14153, Cryptococcus sp. DSM 14154, Rhodotorula yarrowii DSM 14155, Trichosporon mucoides DSM 14156 und Trichosporon dulcitum DSM 14162 zur Detoxifizierung von Mykotoxinen, nämlich Ochratoxinen und/oder Zearalenonen, in Nahrungsmitteln und/oder Futtermitteln, durch Abspaltung der Phenylalaningruppe des Ochratoxins bzw. Abbau des Zearalenons.

8. Verwendung nach Anspruch 7, **dadurch gekennzeichnet, daß** Trichosporon spec. nov. DSM 14153, Eubacterium sp. DSM 14197 oder Stenotrophomonas nitritreducens DSM 14168 zur Detoxifizierung von Mykotoxinen, nämlich Ochratoxinen und/oder Zearalenonen, in Nahrungsmitteln und/oder Futtermitteln eingesetzt sind.

9. Verwendung nach Anspruch 7 oder 8, **dadurch gekennzeichnet, daß** Mischkulturen der Bakterien und/oder Hefen zur Detoxifizierung von Mykotoxinen, nämlich Ochratoxinen und/oder Zearalenonen, in Nahrungsmitteln und/oder Futtermitteln eingesetzt sind.

10. Verfahren zur biologischen Entgiftung von Mykotoxinen, nämlich Ochratoxinen und/oder Zearalenonen, in Nahrungs- und Futtermitteln mit einem Mikroorganismus, **dadurch gekennzeichnet, daß** ein Mikroorganismus nach einem der Ansprüche 1 bis 8, mit den Nahrungs- bzw. Futtermitteln in einer Menge von 0,01 Gew.-% bis 1 Gew.-%, insbesondere 0,05 Gew.-% bis 0,5 Gew.-%, vermischt wird.

11. Verfahren nach Anspruch 10, **dadurch gekennzeichnet, daß** die Nahrungs- oder Futtermittel durch Rühren in einer wäßrigen Suspension des Mikroorganismus, enthaltend 20 bis 99 Gew.-%, insbesondere 35 Gew.-% bis 85 Gew.-% Wasser bei Temperaturen von 10 bis 60 °C, insbesondere 15 bis 45 °C, für 2 min bis 12 h, insbesondere 5 min bis 2 h, behandelt wird.

## Claims

1. A microorganism for the biological detoxification of mycotoxins, namely ochratoxins and/or zearalenons, **characterized in that** a microorganism and, in particular, aerobic or anaerobic detoxifying bacteria or yeasts is/are used, which cleave(s) the phenylalanine group of the ochratoxins and degrade zearalenons, respectively, wherein the mycotoxin-detoxifying bacteria are selected from *Sphingomonas sp.* DSM 14170 and DSM 14167, *Stenotrophomonas nitritreducens* DSM 14168, *Stenotrophomonas sp.* DSM 14169, *Ralstonia eutropha* DSM 14171 and *Eubacterium sp.* DSM 14197, and/or the detoxifying yeasts are selected from *Trichosporon spec. nov.* DSM 14153, *Cryptococcus sp.* DSM 14154, *Rhodotorula yarrowii* DSM 14155, *Trichosporon mucoides* DSM 14156 and *Trichosporon dulcitum* DSM 14162.

2. A microorganism according to claim 1, **characterized in that** the bacteria and/or yeasts are stabilized particularly by lyophilization, spray-drying or microencapsulation.

3. A microorganism according to claim 1 or 2, **characterized in that** the bacteria or yeasts are used as cell-free extracts or crude extracts

4. A microorganism according to claim 3, **characterized in that** said microorganism is used as a cell-free extract in a solution and, in particular, an aqueous solution.

5. A microorganism according to claim 3, **characterized in that** said crude extract is obtained from said bacteria and yeasts by applying ultrasound, enzymatic digestion, a combination of shock-freezing and thawing, a flow homogenizer, a French press, autolysis at a high NaCl concentration, and/or a bead mill.

6. A microorganism according to any one of claims 1 to 4, **characterized in that** said microorganism is used in a buffered solution containing acetate, citrate, phosphate or tris-hydrochloride buffer at a pH of between 1 and 12 and, in particular, 2 and 8.

7. The use of bacteria selected from: *Sphingomonas sp.* DSM 14170 and DSM 14167, *Stenotrophomonas nitritreducens* DSM 14168, *Stenotrophomonas sp.* DSM 14169, *Ralstonia eutropha* DSM 14171 and *Eubacterium sp.* DSM 14197, and/or yeasts selected from: *Trichosporon spec.* nov. DSM 14153, Cryptococcus sp. DSM 14154, Rhodotorula yarrowii DSM 14155, *Trichosporon mucoides* DSM 14156 and *Trichosporon dulcitum* DSM 14162 for the detoxification of mycotoxins, namely ochratoxins and/or zearalenons, in food products and/or animal feeds by cleaving the phenylalanine group of ochratoxin or degrading zearalenon.

8. The use according to claim 7, **characterized in that** *Trichosporon spec.* nov. DSM 14153, *Eubacterium sp.* DSM 14197 or *Stenotrophomonas nitritreducens* DSM 14168 are used for the detoxification of mycotoxins, namely ochratoxins and/or zearalenons, in food products and/or animal feeds.

9. The use according to claim 7 or 8, **characterized in that** mixed cultures of said bacteria and/or yeasts are used for the detoxification of mycotoxins, namely ochratoxins and/or zearalenons, in food products and/or animal feeds.

10. A method for biologically detoxifying mycotoxins, namely ochratoxins and/or zearalenons, in food products and/or animal feeds by the aid of a microorganism, **characterized in that** a microorganism according to any one of claims 1 to 8 is mixed with the food products or animal feeds in amounts ranging from 0.01 % by weight to 1 % by weight and, in particular, 0.05 % by weight to 0.5 % by weight.

11. A method according to claim 10, **characterized in that** said food products or animal feeds are treated by stirring in an aqueous suspension of said microorganism containing water at 20 to 99 % by weight and, in particular, 35 to 85 % by weight, at temperatures of from 10 to 60 °C and, in particular, 15 to 45 °C, for 2 minutes to 12 hours and, in particular, 5 minutes to 2 hours.

## Revendications

1. Microorganisme pour la décontamination biologique de mycotoxines, à savoir d'ochratoxines et/ou de zéaralénones, **caractérisé par** la mise en oeuvre d'un microorganisme, en particulier de bactéries ou de levures détoxiquantes, aérobies ou anaérobies, lequel (lesquelles) dissocie(nt) le groupe phénylalanine des ochratoxines ou décomposent les zéaralénones, les bactéries détoxiquantes en tant que microorganisme étant choisies parmi Sphingomonas sp. DSM 14170 et DSM 14167, Stenotrophomonas nitritreducens DSM 14168, Stenotrophomonas sp. DSM 14169, Ralstonia eutropha DSM 14171 et Eubacterium sp. DSM 14197, et/ou les levures détoxiquantes étant choisies parmi Trichosporon spec. nov. DSM 14153, Cryptococcus sp. DSM 14154, Rhodotorula yarrowii DSM 14155, Trichosporon mucoïdes DSM 14156 et Trichosporon dulcitum DSM 14162.

2. Microorganisme suivant la revendication 1, **caractérisé en ce que** les bactéries et/ou levures sont stabilisées en particulier par lyophilisation, séchage par pulvérisation ou microencapsulage.

3. Microorganisme suivant l'une des revendications 1 et 2, **caractérisé en ce que** les bactéries ou levures sont mises en oeuvre sous forme d'extrait non cellulaire ou d'extrait brut.

4. Microorganisme suivant la revendication 3, **caractérisé en ce que** le microorganisme est mis en oeuvre sous forme d'extrait non cellulaire dans une solution, en particulier une solution aqueuse.

5. Microorganisme suivant la revendication 3, **caractérisé en ce que** l'extrait brut des bactéries et levures est obtenu par ultrason, désagrégation enzymatique, par une combinaison de congélation ultrarapide et de décongélation, homogénéisateur continu, french-press, autolyse pour une concentration NaCl élevée et/ou broyeur à billes.

6. Microorganisme suivant l'une des revendications 1 à 4, **caractérisé en ce que** le microorganisme est mis en oeuvre dans une solution tamponnée, contenant un tampon d'acétate, de citrate, de phosphate ou de tris-hydrochlorure, avec des valeurs de pH comprises entre 1 et 12, en particulier entre 2 et 8.

7. Utilisation de bactéries, choisies parmi : Sphingomonas sp. DSM 14170 et DSM 14167, Stenotrophomonas nitritreducens DSM 14168, Stenotrophomonas sp. DSM 14169, Ralstonia eutropha DSM 14171 et Eubacterium sp. DSM 14197, et/ou de levures choisies parmi : Trichosporon spec. nov. DSM 14153, Cryptococcus sp. DSM 14154, Rhodotorula yarrowii DSM 14155, Trichosporon mucoïdes DSM 14156 et Trichosporon dulcitum DSM 14162, pour la détoxication de mycotoxines, à savoir d'ochratoxines et/ou de zéaralénones, dans des produits alimentaires et/ou fourrages, par dissociation du groupe phénylalanine de l'ochratoxine ou décomposition de la zéaralénone.

8. Utilisation suivant la revendication 7, **caractérisée en ce que** Trichosporon spec. nov. DSM 14153, Eubacterium sp. DSM 14197 ou Stenotrophomonas nitritreducens DSM 14168 sont mis en oeuvre pour la détoxication de mycotoxines, à savoir d'ochratoxines et/ou de zéaralénones, dans des produits alimentaires et/ou fourrages.

9. Utilisation suivant l'une des revendications 7 et 8, **caractérisée en ce que** des cultures mixtes des bactéries et/ou levures sont mises en oeuvre pour la détoxication des mycotoxines, à savoir d'ochratoxines et/ou de zéaralénones, dans des produits alimentaires et/ou fourrages.

10. Procédé de décontamination biologique de mycotoxines, à savoir d'ochratoxines et/ou de zéaralénones, dans des produits alimentaires et fourrages par un microorganisme, **caractérisé en ce qu'**un microorganisme, suivant l'une des revendications 1 à 8, est mélangé à des produits alimentaires ou fourrages dans une quantité de 0,01% en poids à 1% en poids, en particulier de 0,05% en poids à 0,5% en poids.

11. Procédé suivant la revendication 10, **caractérisé en ce que** les produits alimentaires ou fourrages sont traités par agitation dans une suspension aqueuse du microorganisme, contenant 20 à 99% en poids, en particulier 35 à 85% en poids d'eau, à des températures de 10 à 60°C, en particulier de 15 à 45°C, pour 2 minutes à 12 heures, en particulier 5 minutes à 2 heures.
